# EUROPEAN PATENT APPLICATION

(11) **EP 1 004 623 A1**
(43) Date of publication of application: **31.05.2000**
(21) Application number: 99123437.8
(22) Date of filing: 24.11.1999
(51) Int. Cl.: C08K 5/37, C07D 339/08

(54) **Aryl thioacetals useful as stabilizers in polymeric materials**

(30) Priority: 25.11.1998 US 200215
(71) Applicant: GREAT LAKES CHEMICAL CORPORATION, West Lafayette Indiana 47906 (US)
(72) Inventor: Zenner, John M., Stamford, Connecticut 06902 (US)
(74) Representative: Grünecker, Kinkeldey, Stockmair & Schwanhäusser Anwaltssozietät

(57) **Abstract**

The present invention relates to a method of providing antioxidation and imparting thermal and process stability to polymer, as using certain 2-aryl-substituted cyclic dithio compounds.

## Description

The present invention relates to a method of stabilizing polymeric materials using select aryl thioacetals. More particularly, the invention employs specific 2-aryl substituted dithio 5, 6 and 7 member heterocyclic compounds to impart enhanced antioxidant behavior, as well as process and thermal stability to polymers, including polyolefins such as polypropylene.

The invention also relates to the stabilized polymers so obtained; and to certain classes of the dithio compounds used in the practice of the invention.

While long known and continually finding new and manifold uses, commercially important polymers, such as polyolefins, are subject to various forms of degradation. The most deleterious of these involve degradation instigated by thermal exposure and radiation; in certain instances, even the stresses attendant processing, such as extrusion, abet degradation. In polypropylene, for example, attack by oxygen, radiation or excessive heat cause a loss of molecular weight and physical and chemical properties.

Redress of such degradation has been and is the subject of ongoing research efforts. Among the more common solutions devised is the use of chemical additives. Thus: various hindered phenols have been employed as antioxidants; certain phosphites have been used to ameliorate high temperature degradation; and sterically hindered amine light stabilizers have been used to combat degradation caused by light, specifically as caused by uv radiation.

Molecules which incorporate a thioacetal moiety have also been investigated in the context of polymer stabilization. For example, EP 0 389 424 describes hindered amine light stabilizers (HALS) having thioacetal functionality in the form of an ester. U.S. Patent Nos. 4,849,463 and 4,973,619 describe a stabilizer system for polyvinyl chloride (PVC) which comprises primary and co-stabilizers; described therein is a thioglycolic acid ester. JP 50135147 and U.S. Patent No. 3,661,844 report the use of thioacetals as synergistic stabilizers for chlorine-containing polymers, such as PVC. EP 0 349 380 discloses hindered phenolics having thioacetal functionality as stabilizers in polypropylene. Elsewhere, U.S. Patent No. 4,939,185 describes the cyclic thioethers, 1,3-dithiane and 1,4-dithiane, as stabilizers for polycarbonates.

While these additives offer advantages, they also suffer drawbacks and are limited to certain applications. They are also limited in the explicit description of sulfur activity. For example, EP 0389424 and EP0349380 utilize the sulfur linkage to provide additional molecular weight. While the ability of this sulfur linkage to decompose peroxides is assumed, the radical scavenging ability of these molecules is attribuable to the amine and phenol moieties, respectively. Likewise, those utilizing thioacetals do not recognize this as a species capable of acting as a free radical scavenger.

Hence the art recognizes a continuing need to develop new additives for polymers which will attend to the requisite stabilization over a wide range of uses with a minimum of adverse consequences.

In one aspect, the present invention is directed to a method of polymer stabilization which satisfies the foregoing desiderata. The method comprises adding to a polymer, a stabilizingly effective amount of a compound having the formula: 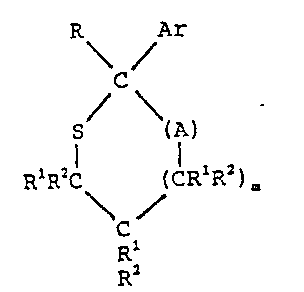 wherein R is hydrogen, lower alkyl or phenyl;
Ar is a monocyclic or fused polycyclic aromatic moiety having up to 15 ring atoms any of which may be unsubstituted or substituted with one or more lower alkyl, lower alkoxy, haloalkyl, nitro, COR^{3,} CO₂R³, 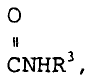 CN, NR₂^{3A} and SO₃R³ groups or Ar can be substituted with one or more Ar groups substituted or unsubstituted as herein defined wherein R³ is hydrogen, halogen or lower alkyl and R^{3A} is hydrogen, lower alkyl, lower
   alkoxy, COR³, CO₂R³ and 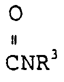 with the proviso that R³ is hydrogen or lower alkyl when the substituent is SO₃R³ and
   when R^{3A} is 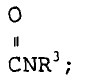
A is 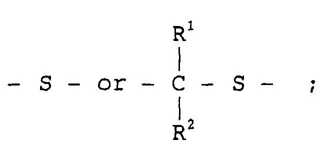
R¹ and R² are each independently hydrogen, lower alkyl, phenyl or R¹ and R² when on adjacent carbon atoms may be joined together with said adjacent carbon atoms to form a 6 membered aromatic ring; and
m is 0, 1 or 2 with the proviso that when A is 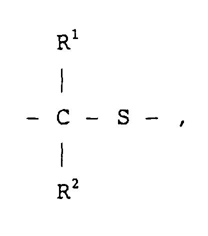 m is 0.

It has been found that the 5 (dithiolanes), 6 (dithianes) and 7 member cyclic dithio compounds of the above formula, where there is aryl substitution at the 2 carbon as further described hereinbelow, offer excellent high temperature process stabilization as well as long term antioxidant behavior.

In another aspect, the present invention is directed to various dithio compounds; and in yet another aspect, the invention is directed to the polymers obtained by virtue of practice of the method aforesaid.

Polymers contemplated by the practice of the instant invention include polyolefins and olefin copolymers; polystyrene and styrenic copolymers; polyamides; polyesters, polycarbonates; and polymers derived from vinyl chloride monomers such as polyvinylchloride (PVC).
FIGURE 1 is a graph depicting oxygen uptake over time for various dithio compounds subject of the present invention where the 2-aryl substituent is monosubstituted.
FIGURE 2 is a graph depicting oxygen uptake over time for various dithio compounds subject of the present invention where the 2-aryl substituent is disubstituted.
FIGURE 3 is a graph depicting oxygen uptake over time for dithiolane and dithiane compounds subject of the present invention.

The 5, 6 and 7 member cyclic dithio compounds contemplated in the practice of the present invention have the formula: 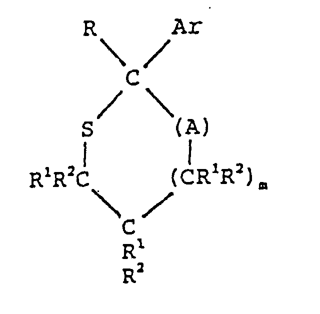 wherein R is hydrogen, lower alkyl or phenyl;
Ar is a monocyclic or fused polycyclic aromatic moiety having up to 15 ring atoms any of which may be unsubstituted or substituted with one or more lower alkyl, lower alkoxy, haloalkyl, nitro, COR³ ,CO₂R³ and 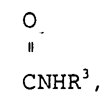 CN, NR₂^{3A} and SO₃R³ groups or Ar can be substituted with one or more Ar groups substituted or unsubstituted as herein defined wherein R³ is hydrogen, halogen or lower alkyl and R^{3A} is hydrogen, lower alkyl, lower
   alkoxy, COR³, CO₂R³ and 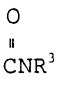 with the proviso that R³ is hydrogen or lower alkyl when the substituent is SO₃R³ and
   when R^{3A} is 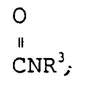
A is 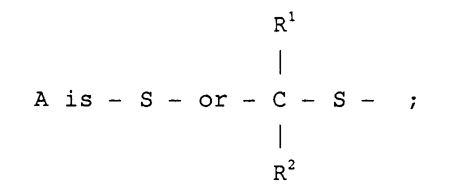
R¹ and R² are each independently hydrogen, lower alkyl, phenyl or R¹ and R² when on adjacent carbon atoms may be joined together with said adjacent carbon atoms to form a 6 membered aromatic ring; and
m is 0, 1 or 2 with the proviso that when A is 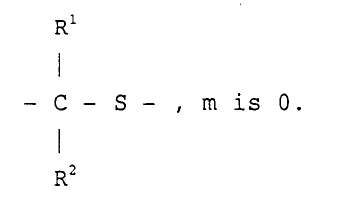 m is 0.

As employed herein, the term "lower alkyl" includes up to C₁₈, preferably to C₁ to C₈ alkyl groups which may be in the normal or branched configuration. Without limitation, examples of such groups include: methyl, ethyl, propyl, isopropyl, butyl, isobutyl, t-butyl, amyl, hexyl, heptyl, octyl, nonyl, decyl, undecyl, dodecyl, tridecyl, tetradecyl, pentadecyl, hexadecyl, heptadecyl and octadecyl.

As employed herein, the term "lower alkoxy" includes up to C₁₈, preferably C₁ to C₈ alkoxy groups which may be in the normal or branched configuration. Without limitation, examples of such groups include methoxy, ethoxy, propoxy, butoxy, pentoxy, hexoxy, heptoxy, octoxy (or octyloxy), nonyloxy, decyloxy, undecyloxy, dodecyloxy, tridecyloxy, tetradecyloxy, pentadecyloxy, hexadecyloxy, heptadecyloxy and octadecyloxy.

Preferred monocyclic aromatic moieties include
5 and 6 member rings such as indenyl and phenyl; fused polycyclic aromatic moieties include ortho-and ortho-and peri-fused systems; ortho-fused moieties, such as α-naphthyl, β-naphthyl, phenanthrenyl and the like are preferred.

As employed herein, the term "haloalkyl" includes lower alkyl groups as hereinbefore defined which have at least one halogen substituent. Full halogen substitution of said groups may also be had. Halogens in this regard include fluorine, chlorine, bromine and iodine. A particularly preferred haloalkyl is trifluromethane.

As employed herein, "nitro" intends the group having the formula NO₂.

In a first embodiment, "A" in the above formula is - S - and subscript m is 1. In the 1,3-dithiane thus contemplated in this embodiment, it is preferred that Ar is a monocyclic aromatic moiety and R¹ and R² are each hydrogen. More preferably, Ar is phenyl and R is also hydrogen, the resulting compound being 2-phenyl-1,3-dithiane.

In a still more preferred practice of this embodiment, said phenyl is substituted with one or more lower alkoxy groups as hereinbefore defined. In this instance it is preferred that said substitutions occur at the 3, 4 and/or 5 positions of the phenyl ring. Particularly preferred compounds in this regard are represented by the following formulae:

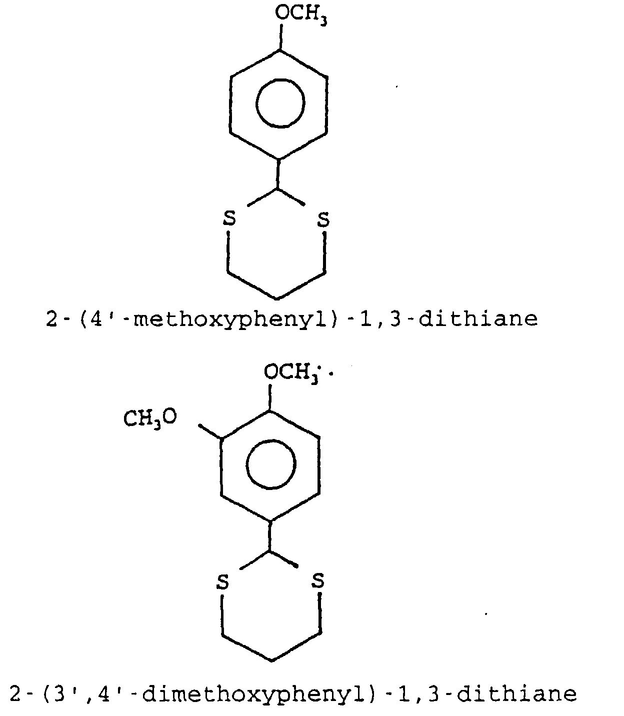

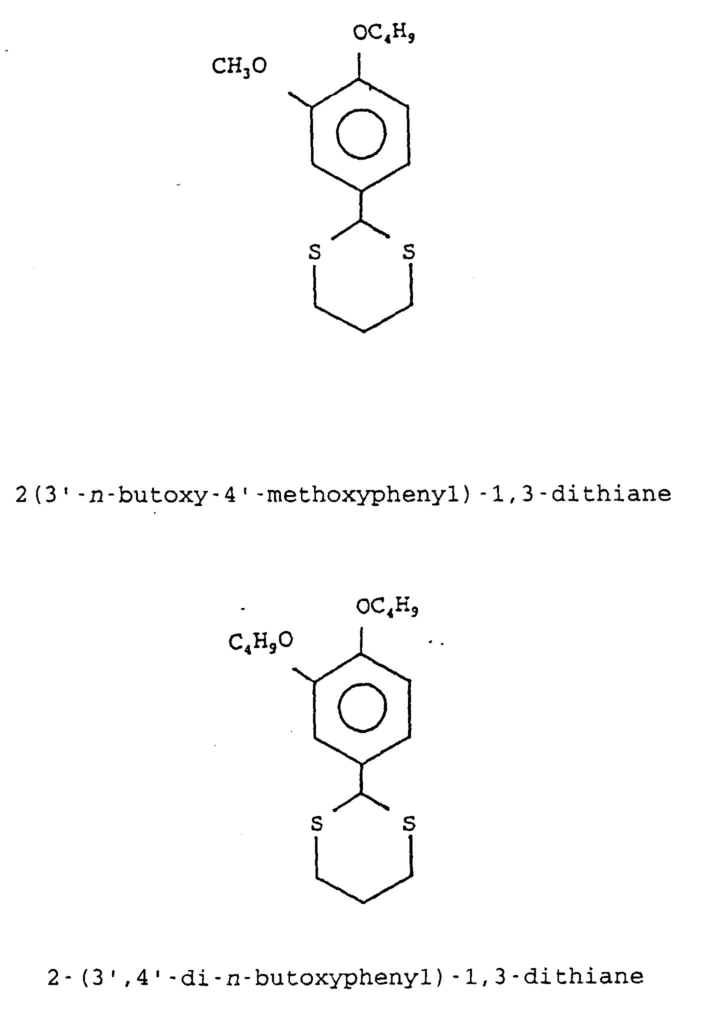

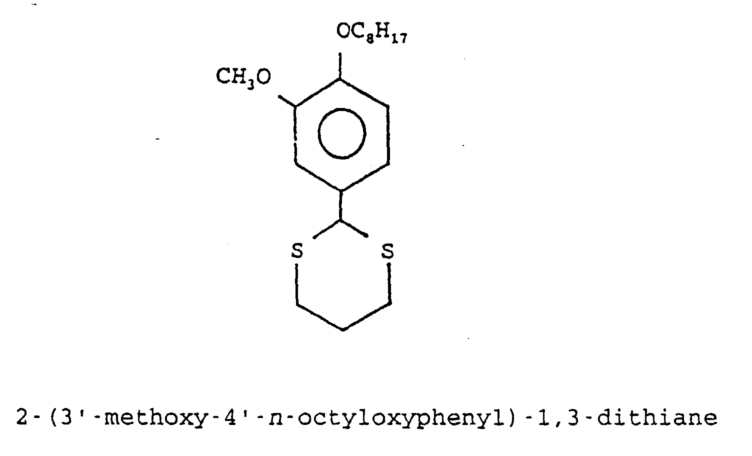

In another aspect of this embodiment, said phenyl ring is substituted with one or more nitro groups. Again, said substitutions preferably occur at the 3, 4 and/or 5 positions of said phenyl ring. Particularly preferred compounds in this regard are represented by the following formula:

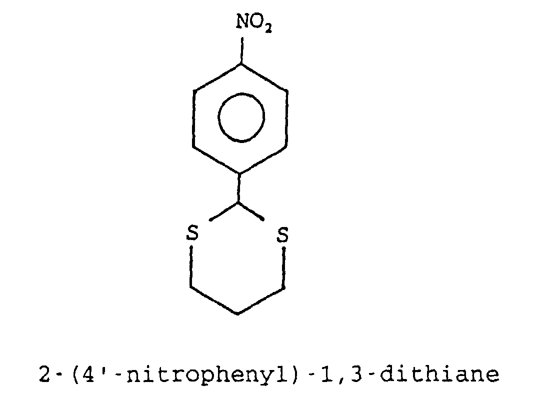

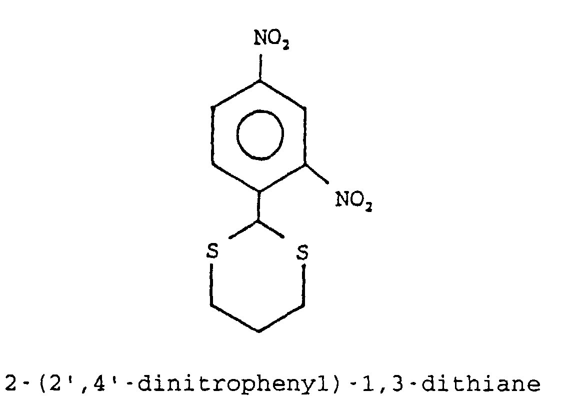

In yet another aspect of this first embodiment, said phenyl ring is substituted preferably at the 3, 4 and/or 5 position with one or more lower alkyl groups or one or more haloalkyl groups. Particularly preferred compounds in this regard are represented by the following formula:

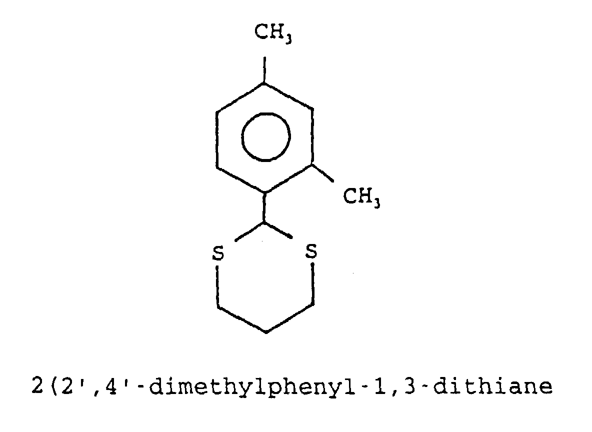

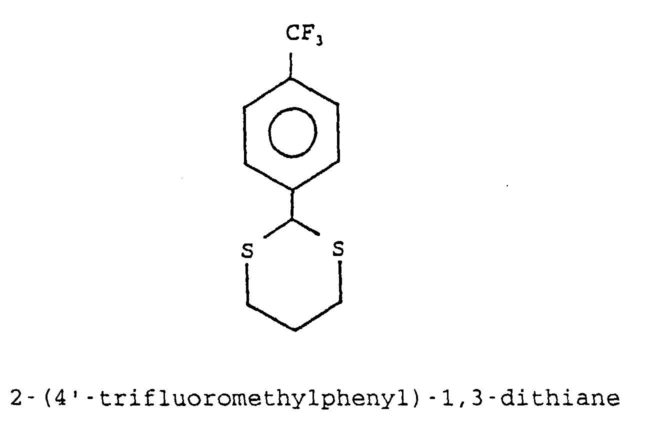

In yet another aspect of the present invention "Ar" is phenyl and R is lower alkyl, preferably a C₁-C₃ alkyl, rather than hydrogen. A representative compound in this regard has the following formula:

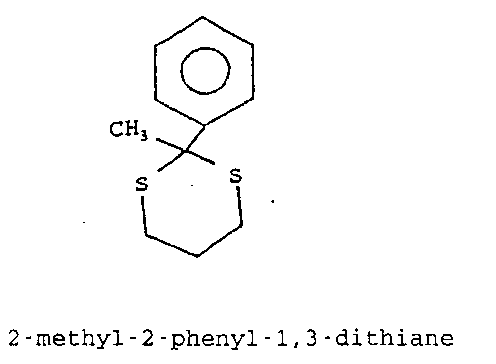

In a second embodiment, "A" in the above formula is -S- and subscript m is O. In the 1,3-dithiolane thus contemplated in this embodiment, it is preferred that Ar is a monocyclic aromatic moiety and R¹ and R² are each hydrogen. More preferably, Ar is phenyl and R is hydrogen. In a still more preferred practice, said phenyl is substituted with one or more lower alkoxy groups as hereinbefore defined. In this instance, it is preferred that said substitutions occur at the 3,4 and/or 5 positions of the phenyl ring.

A particularly preferred compound in this regard is represented by the following formula:

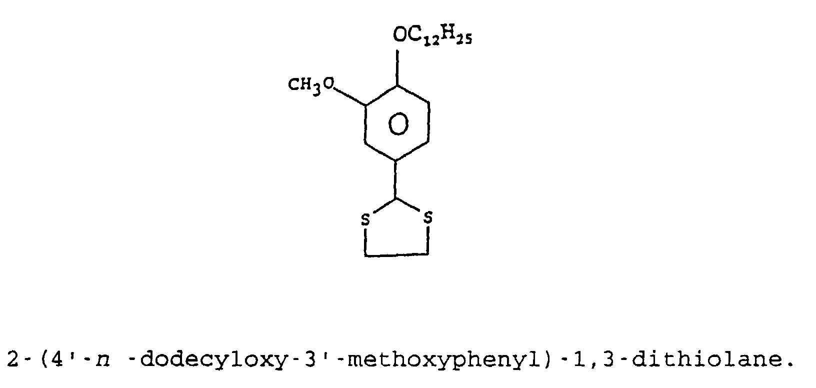

The present invention is also directed to a dithio compound having the following structure: 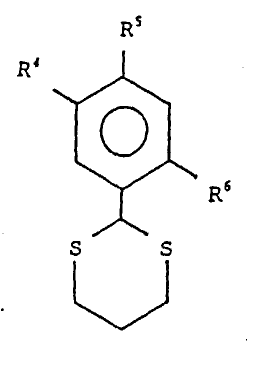 wherein R⁴ is hydrogen or lower alkoxy; R⁵ is lower alkoxy, lower alkyl or nitro; and R⁶ is hydrogen, lower alkyl or nitro, with the provisos that (i) when R⁴ and R⁵ are lower alkoxy, R⁶ is hydrogen; (ii) when R⁵ and R⁶ are lower alkyl or nitro, R⁴ is hydrogen; and (iii) R⁴ and R⁵ are not methoxy at the same time.

In one preferred embodiment R⁴ is methoxy and R⁵ is butoxy. In another, R⁴ and R⁵ are both butoxy. In still another, R⁴ is methoxy and R⁵ is octoxy. In still yet another, R⁵ and R⁶ are each methyl. And in another, R⁵ and R⁶ are each methyl. And in another, R⁵ and R⁶ are each nitro.

The present invention is also directed to a dithio compound having the following structure: 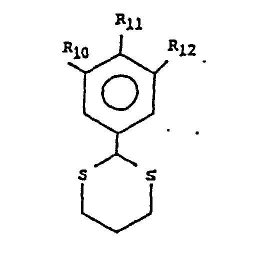 wherein R¹⁰, R¹¹, and R¹² are each independently a lower alkyl, lower alkoxy or CO₂R³ group where R³ is as hereinbefore defined. In a preferred practice R¹⁰, R¹¹, and R¹² are each independently a lower alkyl of C₁ to C₈.

The present invention is further directed to a compound having the formula: 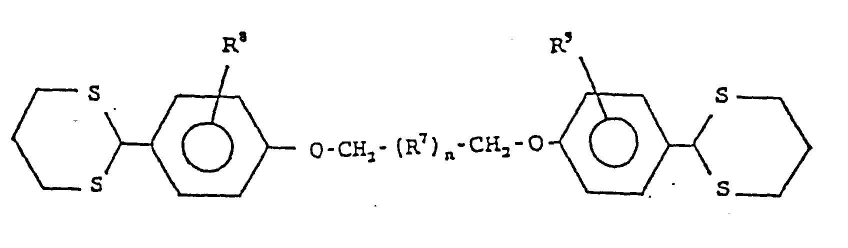 wherein R⁷ is methylene or phenylene and n is 0 or is an integer from 1 to 10 when R⁷ is methylene or an integer from 1 to 12 when R⁷ is phenylene; and R⁸ and R⁹ are each independently hydrogen, lower alkyl or lower alkoxy.

In one embodiment, R⁷ is methylene, R⁸ and R⁹ are each lower alkoxy and n is an integer from 2 to 6. In a first practice of this embodiment, R⁸ and R⁹ are each methoxy and n is an integer from 3 to 5. A preferred compound in this regard has the following structure:

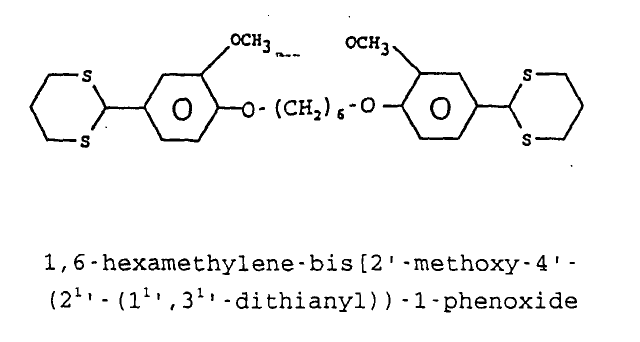

In a another embodiment, R⁷ is phenylene, R⁸ and R⁹ are each lower alkoxy and n is an integer from 1 to 12. In a first practice of this embodiment, R⁸ and R⁹ are each methoxy and n is an integer from 1 to 4; most preferably n is 1. A preferred compound in this regard has the following structure:

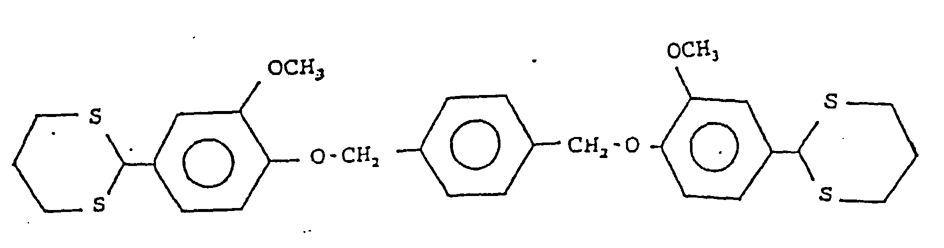

In practice, the compounds employed in the present inventive method may be added to the polymer using conventional techniques, such as via extrusion.

Without limitation, polymers contemplated in regard to the subject invention include polyolefins and olefin copolymers, such as e.g. polyethylene including polyethylene homopolymers and copolymers, including high density and linear low density polyethylene; elastomers of polyethylene with other polyolefins; and most especially polypropylene. Other polymers contemplated in regard to the practice of the invention are polystyrene and styrenic copolymers, polyamides, polycarbonates, polyesters and polymers derived from vinyl chloride monomers, such as PVC.

In practice, the dithio compounds of the present invention are added to the polymer in an amount of from about 0.01 to about 5 weight percent based upon the weight of said polymer. Preferably, the amount is from about 0.01 to about 0.5 weight percent. Most preferably, about 0.05 to about 0.25 weight percent.

The compounds of the present invention may also be employed in conjunction with other additives known heretofore, such as those including hindered phenols, e.g. tetrakis(methylene[3,5-di-t-butyl-4-hydroxy-hydrocinnamate])methane, as commercially available under the tradename Anox 20; or phosphites, e.g. tris(2,4-di-t-dibutylphenyl) phosphite commercially available under the tradename Alkanox 240; hindered amines, e.g. bis-(2,2,6,6-tetramethyl-4-pieridinyl) sebacate; and 2-hydroxybenzophenones and 2-(2'-hydroxyphenyl) benzotriazoles. Also contemplated in this regard are aryl substituted benzofuranones; preferably, substituted 3-aryl-2(3H)-benzofuranones.

A particulary preferred benzofuranone having this formula is 5,7-bis(1,1-dimethylethyl)-3-(3,4-dimethylphenyl)-2(3H)benzofuranone,

The following examples are offered to illustrate aspects of the invention and are not limitations on the scope thereof.

### EXAMPLES

Representative dithianes subject of the present invention were prepared as follows:

**Preparation of Dithianes:** All dithianes were prepared from the corresponding benzaldehydes using an adaptation of a procedure outlined in Greene, T. W. et al. "Protective Groups in Organic Synthesis", Second Edition, John Wiley & Sons, Inc., New York, NY (1991), Page 201. Specifically: to a 50 mL 3-neck flask was added 10 mmol of arylaldehyde (corresponding to the compounds delineated below), 15 mL of CH₂Cl₂ (dried over molecular sieves). The system was purged with N₂ and then 20 mmol of either 1,3-propanedithiol or 1,2-ethane dithihol was added. 20 mmol of BF₃·OEt₂ was then slowly added via a syringe dropwise with vigorous stirring. The reaction was to varying extents exothermic, depending upon the substrate. A cold water bath was kept on hand to provide a heat sink for this reaction if necessary. The reaction was stirred at room temperature for 2-3 hours then diluted with Et₂O, washed with deionized water (DIW) (2x) and saturated NaCl, and dried over Na₂SO₄. Removal of the solvent afforded a solid (usually pale yellow) which was recrystallized from Et₂O or THF with a small amount of hexanes added to precipitate. Yields for dithianes ranged from 71 to 90 percent.

Prepared using this method were the following 2-aryl-1,3-dithianes. Melting points and TGA's, where available, are provided.

**2-(4'-methoxyphenyl)-1,3-dithiane:** Obtained as a white solid in 77% yield. mp 112-114.5°C.

**2-(4'-nitrophenyl)-1,3-dithiane:** Obtained as a yellow solid in 80% yield. mp 139-140.5°C.

**2-(4'-trifluoromethylphenyl)-1,3-dithiane:** Obtained as a white crystalline solid in 71% yield. mp 100.5-102°C.

**2-(2',4'-dinitrophenyl)-1,3-dithiane:** Obtained as a yellow crystalline solid in 83% yield. mp 131-132°C.

**2-(2',4'-dimethylphenyl)-2,3-dithiane:** Obtained as a very pale pink crystals in 88% yield. mp 132-133°C.

**2-(3',4'-dimethoxyphenyl)-1,3-dithiane:** Obtained as a white crystalline solid in 85% yield. mp 90.4-91°C, TGA (5% mass loss) 182°C.

**2-methyl-2-phenyl-1,3-dithiane:** Obtained as a white solid in approximately 50% yield.

**2-(4'-*n*-butoxy-3'-methoxyphenyl)-1,3-dithiane:** Obtained as a white solid in 87% yield. mp 88.5-90°C, TGA (5% mass loss) 201°C.

**2-(3',4'-di-*n*-butoxyphenyl)-1,3-dithiane:** Prepared from 2-(3'4-dimethoxyphenyl)-1,3-dithiane, as above, by treating first with BBr₃ in CH₂Cl₂ to afford 2-(3',4'-dihydroxyphenyl)-1,3-dithiane in 88% yield. This was then treated with NaH in THF followed by *n*-butylbromide to give the desired product in moderate yield. mp 56.2-57.2°C, TGA (5% mass loss) 217°C.

**2-(3'-methoxy-4'-*n*-octyloxyphenyl)- 1,3-dithiane:** Recrystallized from CH₂Cl₂/MeOH. Obtained as a white solid in 87% yield. mp 59-61°C TGA (5% mass loss) 227°C.

**1,6-Hexamethylene-bis[2'-methoxy-4'-(2'*'*-(1'*'*,3''dithianyl))-1-phenoxide]:** Recrystallized from CHCl₃/Et₂O to afford a yellow solid in 91% yield.

**α,α-1,4-Xylene-bis[2'-methoxy-4'-(2'*'*-(1'*'*,3'*'*-dithianyl))-1-phenoxide]:** Recrystallized from toluene to afford a white solid in 39% yield.

**Alkylation of Phenols:** The alkylation of vanillin and other phenols was carried out in the following manner. To a round bottom flask with stir bar was added 25 mmol vanillin, 25 mL of 1:1 DMF/THF, 27 mmol NaOH and 27 mmol of a primary alkylbromide. The solution was brought to reflux and monitored by TLC and GC until the starting material was consumed (or nearly consumed). Typically this reaction did not go to completion and a significant amount of starting material remained unreacted. After cooling to room temperature the reaction mixture was poured into hexanes, washed with DIW (1x), 2.5% NaOH (2x), then again with DIW (1x). The organic phase was then dried over MgSO₄ and concentrated to give a light brown oil which crystallized from hexanes to give a white solid.

### EXAMPLE 1

The oxygen uptake of various compounds employed in the present invention was measured. This parameter is indicative of antioxidant behavior.

Oxygen uptake was measured using, as a model system, the oxidation of 1,2,3,4-tetrahydronaphthalene (tetralin) using 2,2'-azobisisobutyronitrile (AIBN) as a free radical initiator. This technique relates the rate of oxidation of the substrate to oxygen consumption, as measured as a drop in pressure in a closed system. The protocol was as follows:

**Oxygen Uptake:** Oxygen uptake was measured using a modified procedure described in Klemchuk, P. P. et al. Makromol. Chem., Macromol. Symp. (1989) 28:117-144. To a 100 mL 3 neck flask was added 0.24 mmol of various dithiol compounds contemplated by the invention and 39 mL of 2.5 M tetralin in PhCl. A convection pressure gauge attached using a kajon adapter was fitted to one neck of the flask. Through another neck of the flask oxygen was purged through the solution while the third neck was vented through an oil trap (approximately 5 minute purge). The system was then sealed with glass stoppers and the temperature and pressure allowed to equilibrate at 60°C for approximately 15 minutes. After equilibration, 1.13 mmol of AIBN was quickly transferred in 2 mL of 2.5 M tetraline/PhCl and the system resealed. The pressure within the closed system was then monitored versus time using the convention gauge.

Control runs using no additive (denominated as "control") and using conventional distearyl 3,3'-thiopropionate (denominated as "DSTDP") as an additive were also conducted.

In a first set of experiments, various dithianes where the 2-aryl was unsubstituted and monosubstituted as contemplated by the invention were measured. These were:

| **RUN** | **DITHIO COMPOUND** |
|---|---|
| 1 | 2-phenyl-1,3-dithiane |
| 2 | 2-(4'-methoxyphenyl)-1,3-dithiane |
| 3 | 2-(4'-nitrophenyl)-1,3-dithiane |

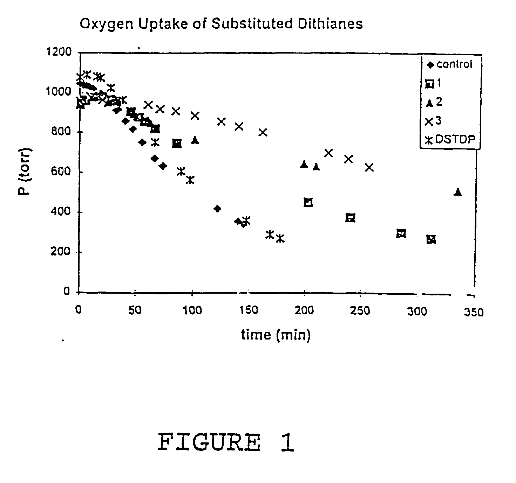

The results are depicted in Figure 1. Figure 1 is a plot of pressure drop "P" (in Torr) over time which correlates to oxygen uptake attributable to polymer oxidation. As seen, the dithianes of Runs 1, 2 and 3 all measurably slowed oxygen uptake relative to the controls using either no additive or using DSTDP, thus indicating antioxidant behavior.

### EXAMPLE 2

Example 2 employed the oxygen uptake protocol of Example 1, but used the following disubstituted dithianes (Runs 5, 6 and 7) and a dithiane having a methyl in place of a benzylic hydrogen (Run 8).

| **RUN** | **DITHIO COMPOUND** |
|---|---|
| 5 | 2-(2',4'-dimethylphenyl)-1,3-dithiane |
| 6 | 2-(2',4'-dinitrophenyl)-1,3-dithiane |
| 7 | 2-(3',4'-dimethoxyphenyl)-1,3-dithiane |
| 8 | 2-methyl-2-phenyl-1,3-dithiane |

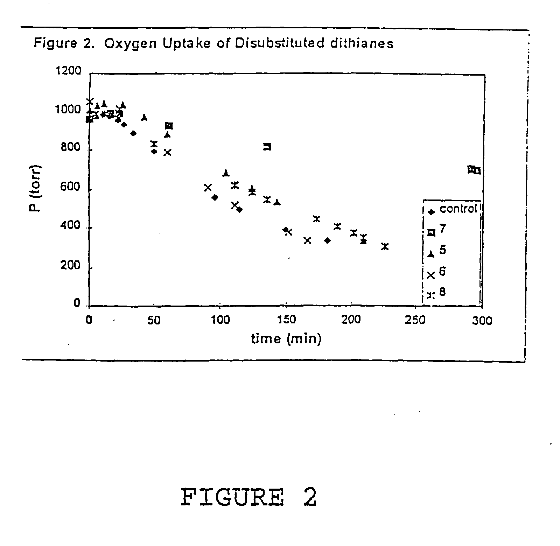

Again, the controls employed no additive and used DSTDP, respectively. The results are depicted in Figure 2. As seen, all the dithianes showed antioxidant activity. Also as apparent the meta positioning of the dithiane in Run 7 showed increased antioxidant activity versus the ortho-substituted compounds of Runs 5 and 6.

### EXAMPLE 3

This example demonstrates the effect of various compounds subject of the invention in offering process stability to polypropylene under conditions of high shear. In this example, several low molecular weight dithianes were incorporated into polypropylene (PP) using a high speed vertical mixer (turbo mixer). This preparation is a high shear stress, low temperature, processing technique. Melt flow viscosities were thereafter measured, maintenance of same being indicative of process stability. Melt flow measurements were also taken using Anox 20 and Alkanox 240, separately and together, as controls. The results are shown in Table 1. The protocol was as follows:

**Turbo Mix:** Himont 6501 pp containing 0.1 weight percent calcium stearate (CaSt) was compounded and pelletized using a Killon single screw extruder (temperature profile zone 1 -die: 190, 190, 200, 200°C). The pelletized PP was dry mixed with additives and then placed in a high speed vertical blender. Two mixing cycles of 26 s (right/left) was usually sufficient to form molten polymer. In the event adequate mixing was not achieved, 10 s mixing intervals were carried out until the polymer was molten. The molten sample was then rapidly applied to a two-roll mill at 65°C to produce an approximately 0.5 mm tape.

**Melt Flow Index:** Melt flow rate was measured using a Tinius Olsen Extrusion Plastometer model MP 993. The unit is a dead-weight piston plastometer with which the extrusion rate of thermoplastic materials through an orifice is determined under prescribed conditions of temperature (230°C) and pressure (2160 g, 6.35 mm piston travel), 360 s preheat time, 4.0 ± 0.2 g, in accordance with ASTM Standard Test Method D1238.

**TABLE 1**

| Melt Flow Data for PP Processed using a 'Turbo Mixer' | | | | | | |
|---|---|---|---|---|---|---|
| RUN | DITHIO COMPOUND 2-ARYL-1,3-DITHIANE | WT. % | WT. % ANOX 20 | WT. % ALKANOX 240 | WT. % CaSt | MELT FLOW (g/10 min.) |
| 1 | None | - | 0 | 0 | 0.1 | 50.0 |
| 2 | 2-(4'-nitrophenyl)-1,3-dithiane | 0.4 | 0 | 0 | 0.1 | 8.0 |
| 3 | 2-(3',4'-dimethoxyphenyl)-1,3-dithiane | 0.4 | 0 | 0 | 0.1 | 6.4 |
| 4 | 2-(3',4'-dimethoxyphenyl)-1,3-dithiane | 0.15 | 0 | 0.15 | 0.1 | 7.7 |
| 5 | None | - | 0.15 | 0.15 | 0.1 | 5.1 |
| 6 | None | - | 0.30 | 0 | 0.1 | 7.1 |

As evident, melt flow viscosities were maintained in Runs 2, 3 and 4 in accordance with the present invention, with Runs 3 and 4 giving improved melt flow performance and better color and odor properties than Run 2.

### EXAMPLE 4

Higher molecular weight dithianes subject to the invention were evaluated using a multipass extrusion technique. Incremental increases in molecular weight led to identification of compounds with a TGA high enough to allow extrusion while minimizing superfluous mass.

**TABLE 2**

| TGA Data for Incrementally Higher Molecular Weight Dithianes | |
|---|---|
| **COMPOUND** | **TGA (5% MASS LOSS) (°C)** |
| 2-(3',4'-dimethoxyphenyl)-1,3-dithiane | 182 |
| 2-(3'-*n*-butoxy-4'-methoxyphenyl)-1,3-dithiane | 201 |
| 2-(3',4'-di-*n*-butoxyphenyl)-1,3-dithiane | 217 |
| 2-(3'-methoxy-4'-*n*-octyloxyphenyl)-1,3-dithiane | 227 |

2-(3'-methoxy-4'-*n*-octyloxyphenyl)-1,3-dithiane was evaluated using a multipass extrusion technique as follows.

**Multipass Extrusion:** Multipass extrusion was conducted using a Killon single screw extruder with a temperature profile (Zone 1, 2, 3, die) of 200, 210, 220, 230°C. Formulations utilized in this study are described in Table 4. Himont 6501 polypropylene was dry mixed with the specified amount of these additives and then extruded. Samples of PP were taken after Pass 1, Pass 3 and Pass 5. Melt flow data was obtained from these samples using ASTM Standard Test Method D1238 methodology. Pass 5 polypropylene was then injection molded into plaques. Initial color and gloss measurements were obtained and these plaques were then aged in a convection flow oven at 135°C. Periodic measurement of color and gloss was conducted throughout the study at approximately two week intervals. Weekly to twice weekly observations were made for catastrophic failure.

Samples were taken on Passes 1, 3 and 5. Control runs were performed using calcium stearate Anox 20 and Alkanox 20. The melt flow data for Pass 5 samples are shown in Table 3 below:

**TABLE 3**

| Melt Flow Data From Extrusion Pass 5 of Multipass Evaluation in Polypropylene | | | | | |
|---|---|---|---|---|---|
| **SAMPLE FORMULATIONS** | **WT. % 2-(3'-methoxy-4'-** ***n*****-octyloxyphenyl)- 1,3-dithiane** | **WT. % Anox 20** | **WT. % ALKANOX 240** | **WT. % CaSt** | **MELT FLOW (g/10 min.)** |
| 1 | 0 | 0 | 0 | 0.1 | 15.5 |
| 2 | 0 | .15 | 0.15 | 0.1 | 4.4 |
| 3 | 0 | 0.30 | 0 | 0.1 | 5.0 |
| 4 | 0.30 | 0 | 0 | 0.1 | 6.5 |
| 5 | 0.15 | 0.15 | 0 | 0.1 | 5.1 |

As seen, the use of 2-(3'-methoxy-4'-*n*-octyloxyphenyl)-1,3-dithiane, as representative of the present invention showed significant activity as a process stabilizer. That is, average molecular weight (as correlates to melt flow) was maintained much more effectively than in the control formulation containing only calcium stearate.

### EXAMPLE 5

Thermal stability using the present invention was evaluated. Polypropylene plaques containing 2-(3'-methoxy-4'-*n*-octyloxyphenyl)-1,3-dithiane and 1-6-hexamethylene-bis[2'-methoxy-4'-(2'*'*-(1'*'*,3'*'*-dithianyl))-1-phenoxide] were formed by injection molding as indicated in Example 4. Initial color and gloss were measured using the following techniques:

**Color and Gloss:** Color measurements were made using a Hunter Lab 10°/D65 instrument (Colorquest II) using Universal Software. The indices L*, a*, b*, DE* and YID were recorded. Gloss measurements were taken at a 60° incident angle.

The plaques were aged at 135°C in a convection flow oven and color and gloss measurements taken again. The results are shown at Table 4, with values for DE* and YID given for the initial plaques and as after 696 hours and 1152 hours of aging. Time to catastrophic failure is also noted. The control contained only calcium stearate additive.

**TABLE 4**

| Color Data from Plaques Oven Aging at 135°C | | | | | |
|---|---|---|---|---|---|
| **ENTRY** | *******ADDITIVES (WEIGHT %)** | **INITIAL DE*********;YID** | **696 h DE*********;YID** | **1152 h DE*********;YID** | **CATASTROPHIC FAILURE** |
| smp1 | control | 0.53;4.55 | - | - | 72 h |
| smp2 | Anox 20 (0.15) + Alkanox 240 (0.15) | 0.14;4.90 | 3.98;11.07 | 8.01;18.73 | 5232 h |
| smp3 | Anox 20 (0.30) | 2.50;9.24 | 8.99;20.38 | 14.91;30.93 | 5760 h |
| smp4 | 2- (3'-methoxy-4'-*n*-octyloxyphenyl)-1,3-dithiane (0.30) | 0.20;5.30 | - | - | 264 h |
| smp5 | 2- (3'-metboxy-4'-*n*-octyloxyphenyl)-1,3-dithiane (0.15) + Anox 20 (0.15) | 0.33;5.30 | 4.00;11.27 | 8.25;19.22 | 5256 h |
| smp6 | 2- (3'-methoxy-4'-*n*-octyloxyphenyl)-1,3-dithiane (0.15) + Alkanox 240 (0.15) | 0.66;6.20 | - | - | 408 h |
| smp7 | 2- (3'-methoxy-4'-*n*-octyloxyphenyl)-1,3-dithiane (0.10) + Anox 20 (0.10) + Alkanox 240 (0.10) | 0.10;5.04 | 3.32;10.00 | 6.86;16.76 | 4368 h |
| smp8 | 1,6-hexamethylene-bis [2'-methoxy-4'-(2'*'* -(1'*'*, 3'*'*-dithianyl))-1-phenoxide] (0.30) | 1.20;2.54 | - | - | 24 h¹ |
| smp9 | Irganox HP (Benzolactone) (0.30) | 0.22;4.89 | 4.21;11.48 | 7.58;17.95 | 4608 h |

| | | | | | |
|---|---|---|---|---|---|
| * All formulations included 0.10 weight percent CaSt. | | | | | |
| ¹ Aging discontinued due to surface bloom due to poor solubility of the additive. | | | | | |

As seen, use of 2-(3'-methoxy-4'-*n*- octyloxyphenyl)-1,3-dithiane in Samples 4, 5, 6 and 7 showed improved thermal stability versus the control of Sample 1 wherein no additive was provided. Even greater stability was apparent when use was in conjunction with Anox 20 (Sample 5) and Alkanox 240 (Sample 6), and with a combination of Anox 20 and Alkanox 240 (Sample 7).

In addition to the notable performance in terms of catastrophic failure, the three component system of Sample 7 also showed less color development in long term aging tests as compared to Irganox HP blends (Sample 9) and Anox 20/Alkanox 240 systems (Samples 2 and 3). The initial color of all samples were similar with the exception of formulations containing only Anox 20 which showed significant yellowing. The formulation of Sample 8 also differed in color due to poor solubility of the additive leading to observed bloom after only 24 h at 135°C.

Periodic color and gloss measurements revealed the following trends. Gloss remained essentially unchanged even after 4000 h of aging. Initial color as measured by YID and *DE was improved by the embodiment of the present invention wherein 2-(3'-methoxy-4'-*n*-octyloxyphenyl)-1,3-dithiane was used in conjunction with Anox 20 and Alkanox 240 (Sample 7). This color trend continued in long term aging with the formulation Sample 7 having measurably better color throughout the study.

### EXAMPLE 6

The oxygen uptake of a dithiolane subject of the present invention was measured and compared to dithiane.

The dithiolane employed in this regard was 2-(4'-dodecyloxy-3'-methoxyphenyl)-1,3-dithiolane. It was obtained as a white solid in quantitative yield mp 47.5 to 48.5°C; TGA (5% mass loss) 242°C, from the reaction of 4'-dodecyloxy-3'-methoxybenzaldehyde and 1,2-ethanedithiol using the same procedures described hereinbefore for Preparation of Dithianes.

The dithiane used in this experiment was 2-(3'-4'-dimethoxyphenyl)-1,3-dithiane.

The oxygen uptake protocol described in Example 1 was employed. The control had no additive.

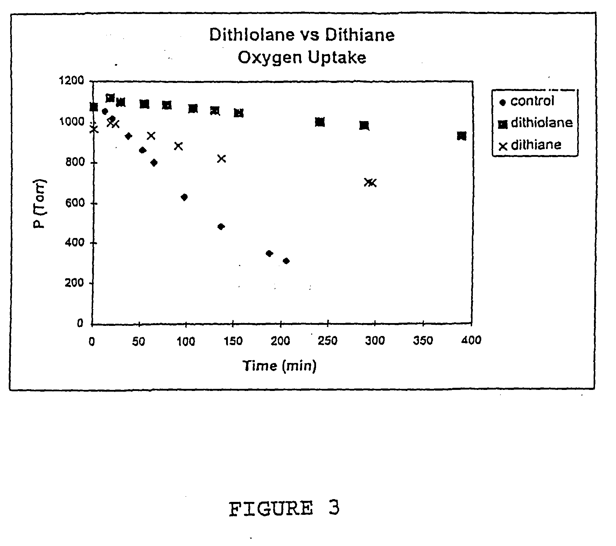

Results are shown in Figure 3. As seen, the dithiolane not only showed improved slowing of oxygen uptake over the control, it also showed greater efficacy than even the dithiane.

### EXAMPLE 7

The effect of the dithiolane of Example 6 on meltflow of polypropylene was evaluated using the same Multipass Extrusion technique described in Example 4, the results were compared to the dithiane of Example 6.

In addition, a comparison of thermal stability using these compounds was measured as described in Example 5.

The results are shown in Table 5, below:

**TABLE 5**

| **Multipass comparison of dithiolane and dithiane followed by oven aging a 135° C.** | | | |
|---|---|---|---|
| | Additive (wt %) | Pass 5 melt flow | Catastrophic failure |
| control | none | 15.5 | 48h |
| 2-(4'-dodecyloxy-3'-methoxyphenyl)-1-3-dithiolane | 0.30 | 6.0 | 2376h |
| 2-(3'-4'-dimethoxyphenyl)-1,3 -diathiane | 0.30 | 6.5 | 264h |

As seen from the Pass 5 data, the use of 2-(4'-dodecyloxy-3'-methoxyphenyl)-1,3 dithiolane, as representative of this class of compounds in the practice of the present invention, showed significant activity as a process stabilizer, and indeed performed slightly better than the dithiane.

Also as can be seen, in long term heat aging the dithialane afforded excellent thermal stability to the polypropylene as compared to the control formulation using no additive and as Compared to the dithiane.

### EXAMPLE 8

**Preparation of Dithiepanes and Benzodithiepins.** The formation of 7- metered ring thioacetal derivatives comtemplated by the present invention are made using the same synthetic methodology as set forth hereinbefore. 1,4-Butanedithiol is reacted with an appropriate aryl aldehyde or ketone in the presence of a lewis acid to afford 2-aryl-1,3-dithiepane. 1,5-dihydro-3-aryl-2,4-benzodithiepins are readily prepared from the acid catalyzed condensation of 1,2-benzenedimethane and an appropriate aldehyde or ketone. Ref: Greene, T.W. and Wuts, P.G.M. Protective Groups in Organic Synthesis.

## Claims

1. A method of polymer stabilization which comprises adding to a polymer a stabilizingly effective amount of a compound having the formula: 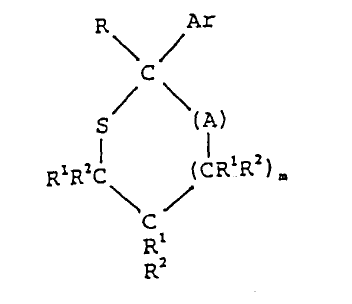 wherein R is hydrogen, lower alkyl or phenyl;
Ar is a monocyclic or fused polycyclic aromatic moiety having up to 15 ring atoms any of which may be unsubstituted or substituted with one or more lower alkyl, lower alkoxy, haloalkyl, nitro and COR³, CO₂R³, 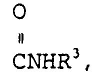 CN, NR₂^{3A} and SO₃R³ groups or Ar can be substituted with one or more Ar groups substituted or unsubstituted as herein defined wherein R³ is hydrogen, halogen or lower alkyl and R^{3A} is hydrogen, lower alkyl, lower
alkoxy, COR³, CO₂R³, and 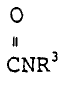 with the proviso that R³ is hydrogen or lower alkyl when the substituent is SO₃R³ and
when R^{3A} is 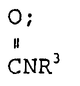
A is 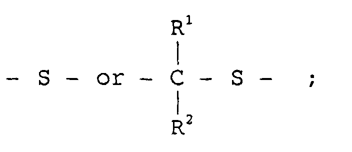
R¹ and R² are each independently hydrogen, lower alkyl, phenyl or R¹ and R² when on adjacent carbon atoms may be joined together with said adjacent carbon atom to form a 6 membered aromatic ring; and
m is 0, 1 or 2 with the proviso that when A is 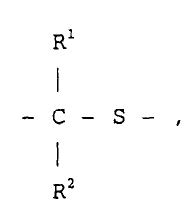 m is 0.

2. The method of Claim 1 wherein A is - S - and m is 1.

3. The method of Claim 2 wherein Ar is a monocyclic aromatic moiety and R¹ and R² are each hydrogen.

4. The method of Claim 3 wherein Ar is phenyl and R is hydrogen.

5. The method of Claim 4 wherein said phenyl is substituted with one or more lower alkoxy groups.

6. The method of Claim 5 wherein said compound has the formula:

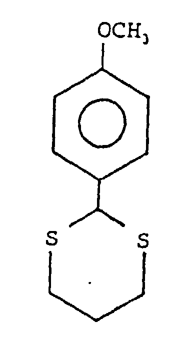

7. The method of Claim 5 wherein said compound has the formula:

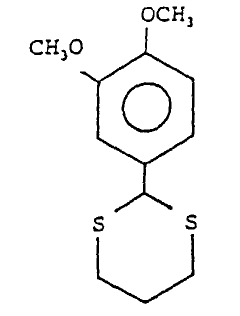

8. The method of Claim 5 wherein said compound has the formula:

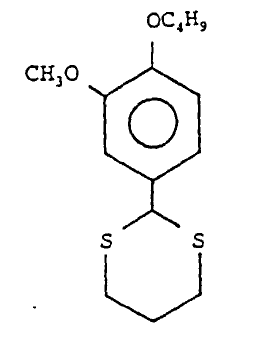

9. The method of Claim 5 wherein said compound has the formula:

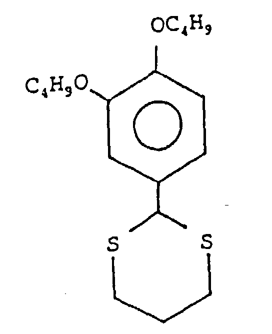

10. The method of Claim 5 wherein said compound has the formula:

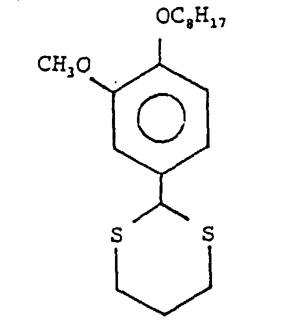

11. The method of Claim 4 wherein said phenyl is substituted with one or more nitro groups.

12. The method of Claim 11 wherein said compound has the formula:

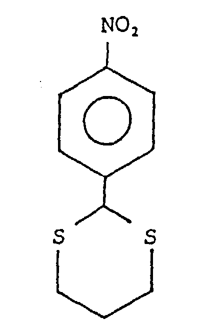

13. The method of Claim 11 wherein said compound has the formula:

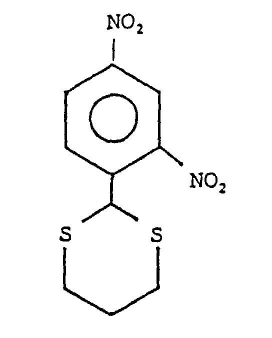

14. The method of Claim 4 wherein said phenyl is substituted with one or more lower alkyl groups.

15. The method of Claim 14 wherein said compound has the formula:

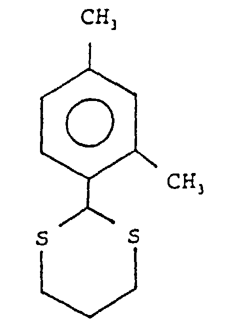

16. The method of Claim 4 wherein said phenyl is substituted with one or more haloalkyl groups.

17. The method of Claim 16 wherein said compound has the formula:

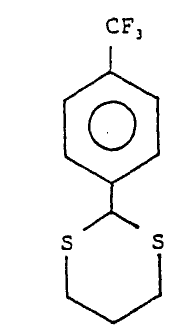

18. The method of Claim 3 wherein Ar is phenyl and R is lower alkyl.

19. The method of Claim 18 wherein said compound has the formula:

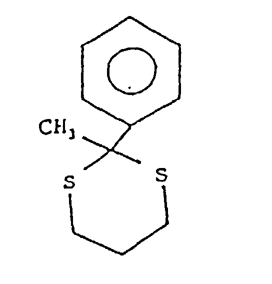

20. The method of claim 2 wherein A is -S-and m is O.

21. The method of Claim 20 wherein Ar is a monocyclic aromatic moiety and R¹ and R² are each hydrogen.

22. The method of Claim 21 wherein Ar is phenyl and R is hydrogen.

23. The method of Claim 22 wherein said phenyl is substituted with one or more lower alkyl groups.

24. The method of Claim 23 wherein said compound has the formula:

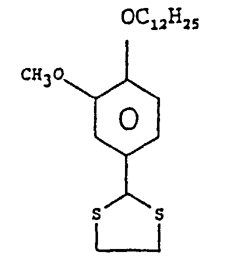

25. The method of Claim 1 wherein said compound is added to said polymer in an amount of from about 0.01 to about 5 weight percent.

26. The method of Claim 25 wherein said amount is from about 0.01 to about 0.5 weight percent.

27. The method of Claim 26 wherein said amount is from about 0.05 to about 0.25 weight percent.

28. The method of Claim 1 wherein said compound is employed with one or more additives selected from the consistency of a hindered phenol, phosphite, hindered amine, 2-hydroxybenzophenonel, 2(-2'- hydroxyphenyl) benzotriazole and an aryl substituted benzofuranonel.

29. The method of Claim 1 wherein said hindered phenol is tetrakis (methylene[3,5-di-t-butyl-4-hydroxyhydrocinnamate, said phosphite is tris (2,4-di-t-butylphenyl) phosphhite; said hindered amine is bis-(2,2,6,6-tetramethyl-4-pierpidinyl) sebacate; and said aryl substituted benzofuranone is a substituted 3-aryl-2(3H)-benzofuranone.

30. The method of Claim 29 wherein said substituted 3-aryl-2(3H) beuzofuranone is 5,7-bis(1,1-dimethylethyl)-3-(3,4-dimethylphenyl)-2(3H) benzofuranone.

31. The polymer product obtained by the method of Claim 1.

32. The polymer product of Claim 31 wherein said polymer is selected from the group consisting of a polyolefin, an olefin copolymer, polystyrene, a styrenic copolymer, a polyamide, a polyester, a polycarbonate and a polymer derived from a vinyl chloride monomer.

33. The polymer product of Claim 32 wherein said polyolefin is polypropylene.

34. A compound having the formula: 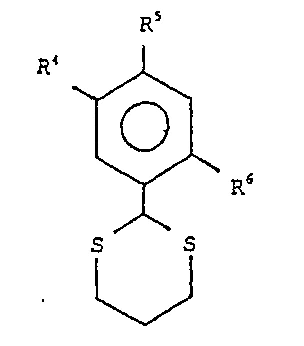 wherein R⁴ is hydrogen, lower alkoxy; R⁵ is lower alkoxy, lower alkyl or nitro; and R⁶ is hydrogen, lower alkyl or nitro with the provisos that (i) when R⁴ and R⁵ are lower alkoxy, R⁶ is hydrogen; (ii) when R⁵ and R⁶ are lower alkyl or nitro, R⁴ is hydrogen; and (iii) R⁴ and R⁵ are not methoxy at the same time.

35. The compound of Claim 34 wherein R⁴ is methoxy and R⁵ is butoxy.

36. The compound of Claim 34 wherein both R⁴ and R⁵ are butoxy.

37. The compound of Claim 34 wherein R⁴ is methoxy and R⁵ is octoxy.

38. The compound of Claim 34 wherein R⁵ and R⁶ are each methyl.

39. The compound of Claim 34 wherein R⁵ and R⁶ are each nitro.

40. A compound having the formula: 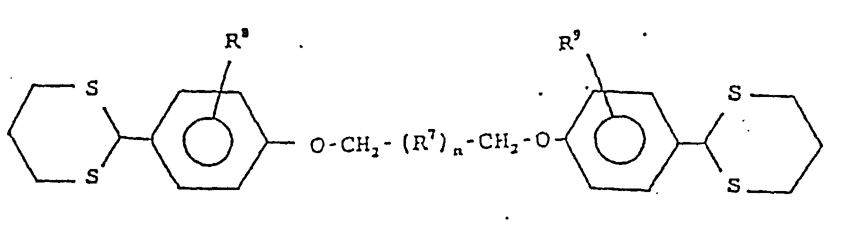 wherein R⁷ is methylene or phenylene and n is 0 or is an integer from 1 to 10 when R⁷ is methylene or an integer from 1 to 12 when R⁷ is phenylene; and R⁸ and R⁹ are each independently hydrogen, lower alkyl or lower alkoxy.

41. The compound of Claim 40 wherein R⁷ is methylene, R⁸ and R⁹ are each lower alkoxy and n is an integer from 2 to 6.

42. The compound of Claim 40 wherein R⁸ and R⁹ are each methoxy and n is an integer from 3 to 5.

43. The compound of Claim 40 having the formula:

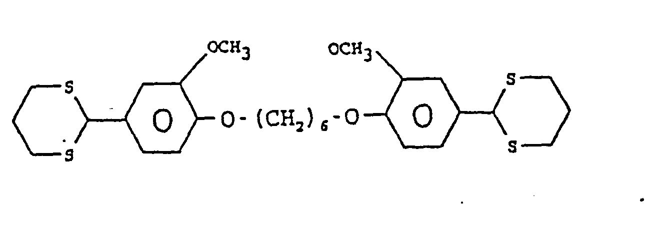

44. The compound of Claim 43 wherein R⁷ is phenylene, R⁸ and R⁹ are each lower alkoxy and n is an integer from 1 to 12.

45. The compound of Claim 44 wherein R⁸ and R⁹ are each methoxy and n is 1 to 4.

46. The compound of Claim 44 having the formula:

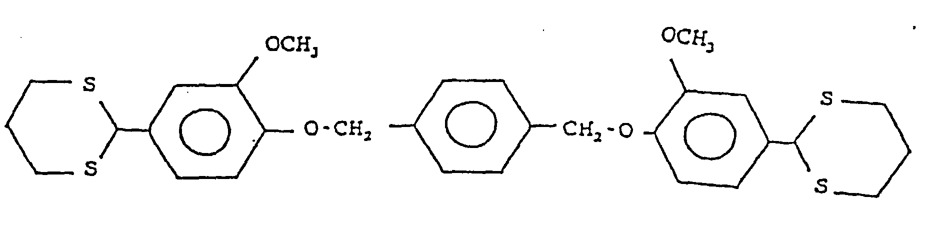

47. A compound having the formula: 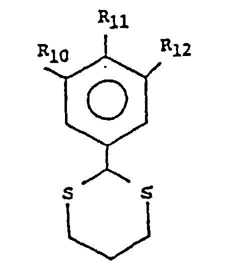 wherein R¹⁰, R¹¹ and R¹² are each independently a lower alkyl, lower alkoxy or CO₂R³ group above R³ is hydrogen, halogen or lower alkyl.

48. The compound of Claim 47 wherein R¹⁰, R¹¹ and R¹² are each independently a lower alkyl of C₁ to C₈ or a lower alkyl of C₁ to C₈.
